(19) Europäisches Patentamt
European Patent Office
Office européen des brevets

(11) Numéro de publication: **0 022 407**
**B1**

(12) **FASCICULE DE BREVET EUROPEEN**

(45) Date de publication du fascicule du brevet: **21.04.82**

(51) Int. Cl.³: **C 07 F 9/65, A 61 K 31/675**

(21) Numéro de dépôt: **80401002.3**

(22) Date de dépôt: **02.07.80**

(54) **Cyclophosphathiazènes, procédé pour leur préparation et compositions les contenant.**

(30) Priorité: **04.07.79 FR 7917336**

(43) Date de publication de la demande:
**14.01.81 Bulletin 81/2**

(45) Mention de la délivrance du brevet:
**21.04.82 Bulletin 82/16**

(84) Etats contractants désignés:
**AT BE CH DE FR GB IT LI LU NL SE**

(56) Documents cités:
**EUROPEAN JOURNAL OF CANCER, vol. 15, nº 5, 1979 GB J. F. LABARRE et al.: "Antitumour Activity of Some Cyclophosphazenes", pages 637—43**

**THE MERCK INDEX, 9 édition, 1976, page 1241, ref. 9346 Rahway N.J. US & J. Am. Chem. Soc. 77, 5915 (1955) WYSTRACH et al.: "Triethylenemelamine"**

(73) Titulaire: **ANVAR Agence Nationale de Valorisation de la Recherche**
**43, rue Caumartin**
**F-75436 Paris Cedex 09 (FR)**

(72) Inventeur: **Labarre, Jean-François**
**30, rue des Geraniums**
**F-31400 Toulouse (FR)**
Inventeur: **Sournies, François**
**44, Avenue d'Italie**
**F-31000 Toulouse (FR)**
Inventeur: **Van de Grampel, Johan, Prof.**
**Kamperfoelieweg 45**
**NL-9765 HJ Paterswolde (NL)**
Inventeur: **Van der Huizen, Adriaan**
**Willemstraat 57**
**NL-9725 JB Groningen (NL)**

(74) Mandataire: **Corre, Jacques et al,**
**Cabinet Regimbeau 26, Avenue Kléber**
**F-75116 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 022 407 B1

Cyclophosphathiazènes, procédé pour leur préparation et compositions les contenant

La présente invention concerne de nouveaux composés utiles comme médicaments, en particulier dans le traitement des tumeurs.

Les composés selon la présente invention sont apparentés aux dérivés hétérocycliques non-carbonés de type cyclophosphazènes dont un certain nombre ont été déjà dans la littérature.

Parmi les composés de la technique antérieure il faut mentionner les composés de formule:

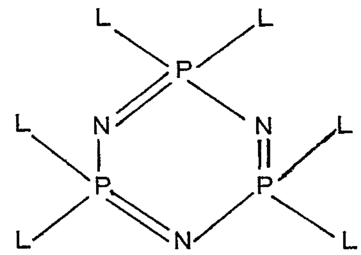

dans laquelle L représente soit le chlore, soit un radical pyrrolyle ou aziridinyle, décrits dans l'article de J. F. LABARRE et coll., Europ. J. Cancer, Vol. 15, pp 637—643, 1979.

Ces composés, en particulier le dérivé dans lequel L est un radical aziridinyle, présentent une activité antitumorale intéressante.

Les recherches effectuées ont conduit à la découverte de nouveaux dérivés hétérocycliques du type cyclophosphathiazène dont le cycle de base est le suivant:

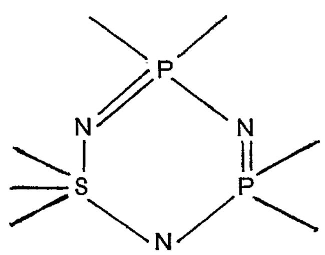

Certains composés de structure cyclophosphathiazénique étaient déjà connus dans la technique antérieure, mais ils ne suggèrent pas la possibilité d'obtenir des composés antitumoraux à partir des composés décrits.

La structure de type cyclophosphathiazène présente comme avantage sur le plan chimique, en particulier par rapport aux dérivés cyclophosphazéniques, de posséder un atome de soufre qui porte de préférence un atome d'oxygène et dont la liaison libre peut être aisément substituée par un grand nombre de radicaux. Cette possibilité de faire varier la nature du substituant de l'atome de soufre permet d'accéder à une famille très vaste de composés actifs ayant comme élément "modulateur d'activité" ce substituant de l'atome de soufre.

Plus particulièrement la présente invention concerne des composés de formule:

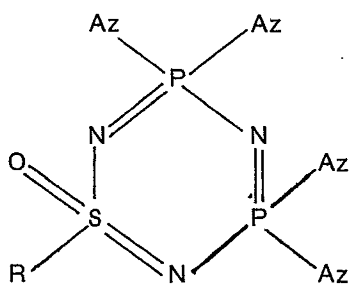

(I)

dans laquelle R est:

— un atome d'halogène,
— un radical alkyle, non substitué ou substitué,
— un radical alcényle, non substitué ou substitué,
— un radical alcynyle, non substitué ou substitué,
— un radical aryle, non substitué ou substitué,

— un radical alkoxy, non substitué ou substitué,
— un radical aryloxy, non substitué ou substitué,
— le radical hydroxy,
— le radical amino, un radical amino N-substitué ou N,N-disubstitué ou les substituants peuvent également former un cycle avec l'atome d'azote.

et Az est un radical 1-aziridinyle éventuellement substitué.

Parmi les composés de formule I particulièrement intéressants, il faut citer les composés de formule:

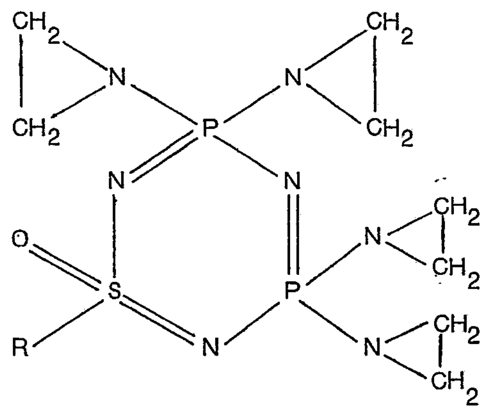

(I')

dans laquelle R a la signification donnée précédemment.

Parmi les substituants du radical 1-aziridinyle Az, il faut citer plus particulièrement les radicaux alkyles et alcoxy.

Par le terme "radical alkyle" utilisé notamment dans la définition de R et pour les substituants du radical Az, il faut entendre plus particulièrement les radicaux alkyles inférieurs, droits ou ramifiés, ayant en particulier de 1 à 7 atomes de carbone comme les radicaux méthyle, éthyle, n-propyle, n-butyle, isobutyle, t-butyle, pentyle et hexyle.

Par le terme "radical alcényle", il faut entendre plus particulièrement les radicaux alcényles inférieurs, droits ou ramifiés, ayant en particulier de 2 à 7 atomes de carbone, comportant une ou plusieurs insaturations éthyléniques comme les radicaux vinyle, allyle, butadiényle par exemple.

Par "radical alcynyle" il faut entendre plus particulièrement les radicaux alcynyles inférieurs, droits ou ramifiés, ayant en particulier de 2 à 7 atomes de carbone, comme les radicaux acétynyle et propargyle.

Les radicaux alkyle, alcényle ou alcynyle selon la présente invention peuvent être non substitués ou substitués, en particulier par des halogènes, par exemple dans le radical trifluorométhyle, ou par un radical aryle, par exemple dans le radical benzyle.

Parmi les radicaux amino N-substitué, il faut citer plus particulièrement les radicaux N-alkyles dans lesquels les radicaux alkyles sont tels que définis précédemment, par exemple les radicaux N-méthylamino, N-éthylamino, N-propylamino, et N-hexylamino.

Parmi les radicaux amino N,N-disubstitué, il faut citer plus particulièrement les radicaux N,N-dialkyles dans lesquels les radicaux alkyles sont tels que définis précédemment, par exemple les radicaux N,N-diméthylamino, N,N-diéthylamino et N-méthyl-N-éthylamino.

Les substituants des radicaux N-substitué et N,N-disubstitué peuvent être différents des radicaux alkyles et en particulier être des radicaux cycloalkyles en $C_3$—$C_6$, par exemple les radicaux N-cyclohexylamino, et N,N-dicyclohexylamino.

Les substituants du radical amino peuvent également former un cycle avec l'atome d'azote, comme par exemple dans les radicaux aziridinyle, pyrrolyle, imidazolyle, pyrazolyle, pyridyle, pyrimidinyle, pyrazinyle, pyrrolidinyle, imidazolidinyle, pipéridyle, et pipérazinyle. Ce cycle peut évidemment être aromatique ou non aromatique et comporter d'autres hétéroatomes que l'atome d'azote, par exemple les radicaux isoxazolyle, furazannyle et morpholinyle; ce cycle peut egalement être substitué, en particulier par un ou des radicaux alkyles ou alkoxy.

Par le terme "radical alkoxy" utilisé notamment dans la définition de R et pour les substituants du radical Az, il faut entendre essentiellement les radicaux alcoxy en $C_1$—$C_7$ correspondant aux radicaux alkyles mentionnés précédemment, méthoxy, éthoxy et butoxy par exemple, et pouvant porter des substituants du même type.

Par "radical aryle" il faut entendre essentiellement des radicaux monocycliques, en particulier le radical phényle, le radical aryle pouvant être substitué de différentes façons, en particulier par un ou plusieurs des radicaux alkyles ou alkoxy définis précédemment, ou halogène, par exemple tolyle, xylyle, éthylphényle, propylphényle, diéthylphényle, bromophényle, chlorophényle et dichlorophényle.

Par radical aryloxy, il faut entendre essentiellement les radicaux aryloxy correspondant aux

radicaux aryl mentionnés précédemment et qui peuvent porter ou non les mêmes substitutions que dans le cas du radical aryle.

Parmi les composés particulièrement préférés selon la présente invention, il faut citer les composés dans lesquels le radical R est un radical phényle, aziridinyle ou un atome de fluor.

La présente invention concerne également un procédé de préparation des composés précédents, dans lequel on fait réagir l'aziridine substitué ou non: Az—H, sur un composé de formule:

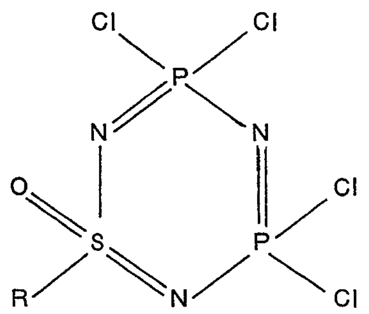

(II)

dans laquelle R a la signification donné précédemment.

Cette réaction est conduite de préférence dans un solvant anhydre, en particulier:
— un éther tel que le diméthyléther, le diéthyléther, le tétrahydrofuranne;
— un hydrocarbure aliphatique tel que l'hexane et l'heptane;
— un solvant aromatique tel que la benzène, le toluène et le nitrobenzène; ou
— un nitrile tel que l'acétonitrile.

La réaction est conduite de préférence à une température comprise entre +100°C et −100°C, de préférence entre +60°C et −90°C et en particulier à une température d'environ −75°C.

La réaction précédente peut être conduite en présence d'un agent accepteur de HCl autre que l'aziridine elle-même, par exemple une amine tertiaire, un hydroxyde alcalin, un carbonate alcalin, un hydroxyde alcalino-terreux ou un hydrogéno carbonate alcalin.

Le rapport molaire des composés en réaction n'est pas un paramètre critique mais on préfère, compte tenu du fait que l'aziridine joue également le rôle d'accepteur d'acide chlorhydrique, travailler en excès d'aziridine substituée ou non par rapport à la stoechiométrie.

Le produit de la réaction de formule I peut être purifié par des procédés connus tels que l'extraction par solvant, la cristallisation, la chromatographie en phase liquide, la chromatographie en phase liquide à haute performance ("HPLC") par exemple.

Dans le cas où le radical R est un radical amino, amino N-substitué ou N,N-disubstitué ou un radical aryloxy ou alcoxy, on peut préparer le composé de formule I correspondant par réaction d'un composé de formule

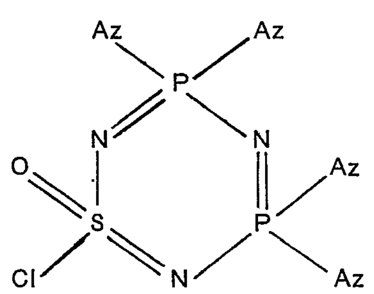

(III)

à l'aide de l'amine respectivement du dérivé hydroxy correspondant au radical R désiré.

La réaction est conduite de préférence à basse température, par exemple inférieure à 20°C.

Dans le cas où le radical R est le radical hydroxy, on peut préparer le composé de formule I correspondant par hydrolyse du composé correspondant de formule III à une température supérieure à 50°C.

La réaction du composé de formule III avec une amine, un dérivé hydroxylé ou l'eau, est de préférence conduite en présence d'un accepteur de HCl tel que mentionné précédemment.

Lorsque le réactif est une amine, un excès de cette amine peut être utilisé comme accepteur de HCl.

Le composé de formule III étant difficile à isoler, il peut être préparé in situ et utilisé sans isolation par exemple à partir d'un composé de formule:

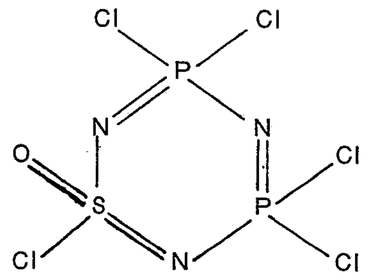

(IV)

par action de l'aziridine.

La réaction du composé de formule IV avec l'aziridine peut être conduite en présence d'un accepteur d'acide, en particulier un excès d'aziridine, par exemple environ 8 moles d'aziridine pour chaque mole de composé IV.

Dans le cas du composé de formule I dans lequel R = Az, celui-ci peut être préparé directement à partir du composé de formule IV par action de l'aziridine en excès dans des conditions identiques à celles indiquées la réaction du composé de formule II sur l'aziridine.

L'excès d'aziridine utilisé dans ce procédé est de préférence un grand excès, par exemple plus de 10 moles d'aziridine par mole de composé IV, de préférence entre 10 et 30 moles d'aziridine pour 1 mole de composé IV.

Le composé de formule IV peut être préparé par des procédés connus, en particulier ceux décrits dans:

— H. H. BAALMANN, H. P. VELVIS, J. C. van de GRAMPEL, Rec. Trav. Chim. (Pays-Bas) *91*, 935—941 (1972).

Les différents produits de départ utiles dans la mise en oeuvre du procédé selon la présente invention peuvent être préparés notamment par les procédés décrits dans:

— H. H. BAALMANN et J. C. van de GRAMPEL, Rec. Trav. Chim. (Pays-Bas) *92*, 1237—1239 (1973),

— H. H. BAALMANN, H. P. VELVIS, J. C. van de GRAMPEL, Rec. Trav. Chim. (Pays-Bas) *91*, 935—941 (1972),

— J. B. van den BERG, B. de RUITER, J. C. van de GRAMPEL, z. Naturforsch. *31b*, 1216—1218 (1976).

Enfin, la présente invention concerne à titre de médicament nouveau notamment en tant qu'agent antitumoral, un composé tel que décrit précédemment ainsi que des compositions pharmaceutiques comprenant à titre de principe actif au moins l'un de ces composés et, en particulier, des compositions pharmaceutiques administrables par voie parentérale.

Parmi les compositions administrables par voie parentérale, il faut citer les compositions injectables, en particulier par voie intrapéritonéale et intraveineuse, bien que l'on puisse utiliser d'autres compositions telles que les pellets. Les produits en cause étant en général assez solubles dans l'eau, on utilisera de préférence comme support pharmaceutique un solvant aqueux, par exemple une solution physiologique de chlorure de sodium. Bien entendu, dans le cas de produits difficiles à solubiliser on peut utiliser des tampons fixant le pH ou éventuellement un solvant non aqueux tel que des esters, des alcools, des polyols ou diverses huiles en combinaison avec des agents émulsifiants, on peut également utiliser des suspensions, en particulier des hydroxypropylcelluloses.

Bien que les compositions pharmaceutiques selon la présente invention soient de préférence des compositions injectables, on peut également utiliser des compositions administrables par voie digestive, voie sublinguale, orale or rectale, ces compositions pouvant se présenter sous forme solide, comprimés, cachets, granulés, gélules, suppositoires par exemple, ou sous forme liquide, gouttes et ampoules.

Dans le cas des compositions par voie orale, celles-ci sont formulées avec des supports connus solides tels que gélatine, gomme arabique, lactose, amidon, polyalkylène glycol, carboxyméthylcellulose par exemple.

Dans le cas de suppositories, on peut utiliser des polyéthylène glycol, de la lanoline à titre de véhicule.

Les compositions selon la présente invention peuvent également être applicables par voie topique, en particulier sous forme de pommade ou de gel applicable sur la peau avec un véhicule inerte tel que la vaseline, les polyéthylène glycol ou d'autres excipients gras; il est possible également de prévoir un agent facilitant la pénétration cutanée du principe actif.

De façon générale, les compositions selon la présente invention peuvent contenir des adjuvants variés tels que conservateurs, stabilisants, mouillants, émulsifiants, agents de texture, de delitement, d'aromatisation, colorants.

Les composés selon la présente invention sont utiles pour le traitement des tumeurs liquides ou solides, par exemple les leucémies, les mélanomes, les sarcomes, les carcinomes. Certains de ces composés se sont révélés particulièrement actifs dans le traitement des leucémies comme le montre les exemples ci-après d'activité sur la leucémie P 388.

La présente invention concerne également un procédé de traitement des tumeurs solides ou liquides qui consiste à appliquer à un patient souffrant d'une telle tumeur une quantité efficace d'au moins un composé selon la formule générale I et en particulier d'un composé selon la formule générale I'.

Les doses appliquées sont, bien entendu, fonction dans chacun des cas du type de tumeur à traiter, ainsi que de l'état général du malade et peuvent varier dans une très large mesure mais sont en général comprises entre 2 et 300 mg/kg par jour, en particulier entre 10 et 300 mg/kg par jour pour les formes injectables, en une ou plusieurs injections dont la fréquence peut être variable et ce suivant la toxicité respective de chacun des produits.

Ainsi, on prévoit des dosages unitaires pour les formes injectables et par voie orale compris entre, de préférence, 50 et 500 mg, en particulier entre 100 et 400 mg de principe actif.

Pour les suppositories on peut prévoir des doses unitaires de 100 à 1000 mg, de préférence 500 à 1000 mg et pour les pommades des concentrations variant entre 1 et 20% en poids.

Les exemples suivants sonts destinés à illustrer le mode de mise en oeuvre de certains des composés selon la présente invention.

Exemple de préparation du produit de départ $(NPCl_2)_2NSOF$

Un mélange de 1,3 g (3,9 mM) de $(NPCl_2)_2NSOCl$ et de 6,5 g (44,6 mM) de $AgF_2$ dans 15 ml de tétrachlorure de carbone sec est agité dans l'obscurité durant 20 heures à la température ambiante. La phase solvant est alors filtrée et évaporée sous pression réduite jusqu'à siccité.

Une sublimation à 40°C sous une pression de 13,3 Pa donne 0,75 g (2,4 mM) de $(NPCl_2)_2NSOF$ pur fondant à 37—38°C (non corrigé), rendement = 61%.

Analyse élémentaire:

| | | | | |
|---|---|---|---|---|
| calculé | N 13,43 | S 10,25 | F 6,07 | Cl 45,33 |
| trouvé | N 13,31 | S 10,18 | F 5,9 | Cl 45,67 |
| | N 13,27 | S 10,07 | F 6,0 | Cl 45,54 |

IR ($cm^{-1}$) (nujol) dans la région de 400—1400 $cm^{-1}$ 498w, 522m, 541vs, 615vs, 672m, 711s, 810s, 878m, 1114w, 1198vs, 1223vs, 1364vs.

Exemple de préparation du produit de départ $(NPCl_2)_2NSOPh$

Un mélange de 2,6 g (7,8 mM) de $(NPCl_2)_2NOCl$ et de 1,05 g (7,8 mM) de chlorure d'aluminium dans 80 ml de benzène est chauffé au reflux sous agitation pendant 48 heures puis refroidi à la température ambiante et versé dans un mélange d'acide chlorhydrique concentré et de glace pilée (rapport en volume 1:7). La phase aqueuse est extraite 2 fois avec 25 ml de benzène et les phases benzéniques sont combinées et lavées à l'eau, séchées sur $CaCl_2$ et évaporées à siccité sous pression réduite.

Le produit est purifié par recristallisation dans le n-pentane. On obtient ainis 4,9 g de $(NPCl_2)_2NSOPh$, rendement = 85%.

Analyse élémentaire:

| | | | | | |
|---|---|---|---|---|---|
| calculé | C 19,43 | H 1,36 | P 16,70 | S 8,64 | Cl 38,23 |
| trouvé | C 19,43 | H 1,38 | P 16,35 | S 8,70 | Cl 38,07 |
| | C 19,33 | H 1,36 | P 16,54 | S 8,79 | Cl 38,13 |

IR ($cm^{-1}$) 470 m, 535 s, 555 s, 602 s, 615 sh, 670 m, 685 m, 695 m, 755 sm, 770 m, 835 s, 870 m, 1000 w, 1024 w, 1100 s, 1187 vs br, 1240 sh, 1272 s.

## Exemple 1

Préparation du composé $(NPAz_2)_2NSOF$

Une solution de 48,0 mmoles d'aziridine fraîchement distillée dans 40 ml de diéthyléther sec est ajoutée goutte à goutte pendant 1 heure à une solution de 3,0 mmoles de $(NPCl_2)_2NSOF$ dans 40 ml de diéthyléther sec, refroidie à −75°C (mélange acétone-azote liquide) sous agitation vigoureuse. On laisse le mélange réactionnel se réchauffer lentement à la température de la pièce et on continue l'agitation pendant encore 17 heures à cette température.

La solution est décantée et le résidu (constitué largement de matière polymère) est extrait trois fois avec des portions de 20 ml de diéthyléther sec.

Après avoir combiné la liqueur-mère et les extraits, le solvant est évaporé sous vide. Le produit de réaction brut est extrait avec trois portions de 80 ml de diéthyléther sec.

La recristallisation par évaporation partielle du solvant et refroidissement subséquent de la

solution conduit à une matière cristalline présentant un point de fusion de 111—112°C, le rendement étant de 62%.

Le composé présente les caractéristiques analytiques suivantes:

Analyse

| | | | | |
|---|---|---|---|---|
| Calculé | C 28,32 | H 4,75 | N 28,90 | S 9,45 |
| Trouvé | C 28,20 | H 4,70 | N 28,75 | S 9,49 |
| | C 28,16 | H 4,72 | N 28,68 | S 9,68 |

IR ($cm^{-1}$) (nujol) dans la région de 400—1400 $cm^{-1}$ 1320s, 1273vs, 1234vs, 1167sh, 1153s, 1122m, 1096m, (960, 942)vs, 881m, 848m, 811m, 777m, 708s, 669s, 650vs, 525m.

Spectre de masse

m/e 339 ($M^+$) 13%, 297 $(M—Az)^+$ 100%

$^{31}P$ NMR (en solution dans $CDCl_3$)

$\delta^{31}P$ 35,7 ppm (par rapport à $H_3PO_4$ 85%) J(P—F) 3,7 Hz.

En utilisant la monométhylaziridine au lieu de l'aziridine, on obtient le composé $[NP(AzCH_3)_2]_2$ NSOF.

## Exemple 2

Préparation du composé $(NPAz_2)_2NSOAz$

Une solution de 90,0 mmoles d'aziridine fraîchement distillée dans 40 ml de diéthyléther sec est ajoutée goutte à goutte pendant 1 heure à une solution de 4,5 mmoles de $(NPCl_2)_2NSOCl$ dans 40 ml de diéthyléther sec, refroidie à −75°C (mélange acétone-azote liquide) sous agitation vigoureuse. On laisse le mélange réactionnel revenir lentement à la température de la pièce puis on agite pendant encore 17 heures à cette même température.

La solution est filtrée et le résidu (constitué essentiellement de matière polymère) est extrait trois fois avec 30 ml de diéthyléther sec.

Après avoir combiné la liqueur-mère et les extraits, le solvant est évaporé sous vide. Le produit de réaction brut est extrait avec trois portions de 80 ml de diéthyléther.

Par recristallisation par évaporation partielle du solvant, refroidissement subséquent de la solution, on obtient une matière cristalline fondant à 86—87°C avec un rendement de 57%.

Ce composé présente les caractéristiques analytiques suivantes:

Analyse

| | | | | |
|---|---|---|---|---|
| calculé | C 33,15 | H 5,56 | N 30,93 | S 8,85 |
| trouvé | C 33,02 | H 5,64 | N 30,53 | S 8,71 |
| | C 33,13 | H 5,68 | N 30,49 | S 8,68 |

IR ($cm^{-1}$) (pastille KBr) dans la région de 400—1400 $cm^{-1}$ 1265s, 1222vs, 1182m, 1151m, 1116m, 1083m, 937vs, 914m, 875s, 842m, 811m, 770s, 710s, 646s, 577m.

Spectre de masse

m/e 362 ($M^+$) 6%, 320 $(M—Az)^+$ 100%.

$^{31}P$ NMR (en solution dans $CDCl_3$)

$\delta^{31}P$ 35,4 ppm (par rapport à $H_3PO_4$ 85%)

Cette préparation conduit parfois à un produit final dont le point de fusion est de 104°C et dont les résultats de l'analyse élémentaire, de la RMN du $^{31}P$ et de la spectrométrie de masse sont identiques à ceux qui viennent d'être décrits à propos de l'échantillon du point de fusion 86—87°C.

Toutefois, les spectres IR (pastille de KBr, région 400—1400 $cm^{-1}$) sont légèrement différents.

1265s, 1226vs, 1170sh, 1153s, 1084s, 942s br, 912m, 882m, 867m, 813m, 766m, 711s, (646, 638)vs, 542m.

Les deux produits ainsi préparés correspondent en fait à deux formes cristallographiques différentes et ont exactement la même activité vis à vis des trois tumeurs décrites ci-dessous.

## Exemple 3

Préparation du composé $(NPAz_2)_2NSOPh$

Un solution de 144,0 mmoles d'aziridine fraîchement distillée dans 40 ml de diéthyléther sec est ajoutée goutte à goutte pendant 1 heure à une solution de 9,0 mmoles de $(NPCl_2)_2NSOPh$ dans 40 ml de diéthyléther sec, refroidie à −75°C (mélange acétone-azote liquide) sous agitation vigoureuse. On laisse le mélange réactionnel revenir lentement à la température de la pièce puis on agite encore pendant 17 heures à cette même température.

La solution est filtrée et le résidue (constitué largement de matière polymère) est extrait trois fois avec des portions de 30 ml de diéthyléther sec.

Après avoir combiné le filtrat et les extraits, le solvant est évaporé sous vide. Le produit de réaction brut est extrait par trois portions de 120 ml de diéthyléther sec.

La recristallisation par évaporation partielle du solvant et refroidissement de la solution conduit à une matière cristalline présentant un point de fusion de 108—109°C avec un rendement de 50%.

Ce composé présente les caractéristiques analytiques suivantes:

Analyse:

| | | | | |
|---|---|---|---|---|
| calculé | C 42,32 | H 5,33 | N 24,67 | S 8,07 |
| trouvé | C 42,74 | H 5,34 | N 24,72 | S 8,19 |
| | C 42,48 | H 5,45 | N 24,56 | S 8,16 |

IR ($cm^{-1}$) (nujol) dans la région 400—1400 $cm^{-1}$ 1267s, 1247vs, 1210 vs br, 1150 vs, 1085s, 939vs br, 878m, 842m, 777m (711, 702)s, 648s, 579s, 534m.
Spectre de masse
m/e 397 ($M^+$) 100%
$^{31}P$ NMR (en solution dans $CDCl_3$)
$\delta$ $^{31}P$ 34,1 ppm (par rapport à $H_3PO_4$ 85%).

Exemple 4

Préparation du composé $(NPAz)_2)_2NSO$ pipéridino

Une solution de 80,0 mmoles d'aziridine fraîchement distillée dans 40 ml d'éther sec est ajoutée à une solution de 10,0 mmoles de $(NPCl_2)_2NSOCl$ dans 40 ml de diéthyléther sec, refroidie à —75°C. Le mélange réactionnel est filtré à —20°C. La solution restante est mise à réagir avec 20 mmoles de pipéridine en solution éthérée à —20°C.

Le $(NPAz_2)_2NSO$ pipéridino est obtenu et identifié par spectre de masse.

En utilisant ce procédé de l'exemple 4 et au lieu de la pipéridine:
— la morpholine,
— le butanol,
— le phénol,
on peut obtenir respectivement:
— le $(NPAz_2)_2NSO$ morpholino,
— le $(NPAz_2)_2NSO$ butoxy,
— le $(NPAz_2)_2NSO$ phénoxy.

Les composés de départ mis en oeuvre dans les exemples précédents peuvent être préparés comme cela est décrit dans les documents suivants ou bien par des procédés analogues:
$(NPCl_2)_2NSOF$ — H. H. Baalmann et J. C. van de Grampel, Rec. Trav. Chim. (Pays-Bas) *92*, 1237—1239 (1973),
$(NPCl_2)_2NSOCl$ — H. H. Baalmann, H. P. Velvis, J. C. van de Grampel, Rec. Trav. Chim. (Pays-Bas) *91*, 935—941 (1972),
$(NPCl_2)_2NSOPh$ — J. B. van den Berg, B. de Ruiter, J. C. van de Grampel, Z. Naturforsch., *31*b, 1216—1218 (1976).

Les composés mentionnés précédemment ont été testés sur le plan de leur activité pharmacologique.

On a tout d'abord observé que la solubilité des trois composés des exemples 1 à 3 est égale ou supérieure à 20 g/l ce qui est donc largement suffisant pour la réalisation de formes injectables en solution aqueuse.

La toxicité de ces composés a été déterminée sur des souris femelles Swiss ou DBA/2.

La léthalité qui apparaît systématiquement les jours 5 et 6 après l'administration a été enregistrée en fonction de la dose inoculée et a permis d'en déduire la dose léthale O qui correspond à la dose maximum non léthale. Les résultats ne dépendent pas de l'espèce de souris utilisée. On a constaté que:
— la dose léthale O du composé $(NPAz_2)_2NSOF$ est de 50 mg/kg,
— la dose léthale O du composé $(NPAz_2)_2NSOAz$ est de 210 mg/kg, et
— la dose léthale O du composé $(NPAz_2)_2NSOPh$ est de 250 mg/kg.

On donne ci-après des exemples de préparations pharmaceutiques comprenant les composés selon la présente invention.

Préparation exemple 1

| | |
|---|---|
| — Composé de l'exemple 2, | 50 mg |
| — Solution saline physiologique, q.s.p. | 10 ml |

Le composé est dissous dans une solution saline et la solution obtenue est mise en ampoule.

Préparation exemple 2
Composition pour une capsule:

| | |
|---|---|
| — Composé de l'exemple 2, | 200 mg |
| — Lactose, | 50 mg |
| — Amidon de pommes de terre, | 50 mg |
| — Cellulose cristallisé, | 109 mg |
| — Stéarate de magnésium, | 1 mg |

Préparation exemple 3
Composition pour 1000 mg de granule:
Les ingrédients suivants sont granulés par des méthodes connues:

| | |
|---|---|
| — Composé de l'exemple 3, | 100 mg |
| — Lactose, | 550 mg |
| — Amidon de maîs, | 330 mg |
| — Hydroxypropylcellulose, | 20 mg |

Préparation exemple 4
Composition pour suppositoires de 1 g:

| | |
|---|---|
| — Composé de l'exemple 2, | 500 mg |
| — Witepsol W-35® (fabriqué par Dynamite Nobel Co.) | 500 mg |

Préparation exemple 5
Composition sous forme de pommade préparée par mélange des ingrédients suivants:

| | |
|---|---|
| — Composé de l'exemple 1, | 2,0 g |
| — Vaseline, | 23,0 g |
| — Alcool stéarylique, | 22,0 g |
| — Propylène glycol, | 12,0 g |
| — Laurylsulfate de sodium, | 1,5 g |
| — p-hydroxybenzoate d'éthyl, | 0,025 g |
| — p-hydroxybenzoate de propyl, | 0,015 g |
| — Eau purifiée, q.s.p. | 100 g |

Activité antitumorale

Les essais d'activité antitumorale ont été conduits sur des souris DBA/2 pour la leucémie P 388 et la leucémie L 1210 et sur des souris Black C 57 pour la mélanome B 16.

Ces essais ont été conduits, soit par monoinjection à différentes doses au jour J + 1 suivant la greffe de la tumeur, soit par poly-injections.

Les résultats observés sont rassemblés dans le tableau I ci-après.

Dans ce tableau:

$$— \text{ILS}(\%) = \frac{T—C}{C} \times 100$$

T (en jour) étant la durée moyenne de survie des souris traitées en jour,
C (en jour) étant la durée moyenne de survie des souris témoins.

— QnD, signifie 1 injection tous les n jours à partir du jour J + 1, ainsi, "3 injections Q4D" signifie
1 injection à J + 1
1 injection à J + 5
1 injection à J + 9

— SOF = composé $(NPAz_2)_2NSOF$
— SOAz = composé $(NPAz_2)_2NSOAz$
— SOPh = composé $(NPAz_2)_2NSOPh$

TABLEAU I

| TUMEUR | PROTOCOLE (par voie i.p.) COMPOSE | DOSE (mg/kg/j) | ILS (%) |
|---|---|---|---|
| | 1 injection J + 1 | 25 | 27 |
| | | 40 | 40 |
| L 1210 | SOF | 50 | 48 |
| | 3 injections Q3D | 25 | 24 |
| | 1 injection J + 1 | 10 | 20 |
| | | 25 | 44 |
| P 388 | SOF | 50 | 98 |
| | 3 injections Q4D | 10 | 51 |
| | | 25 | 118 |
| | 1 injection J + 1 | 100 | 28 |
| | | 150 | 48 |
| L 1210 | SOAz | | |
| | 3 injections Q3D | 50 | 22 |
| | | 100 | 46 |
| | 1 injection J + 1 | 50 | 35 |
| | | 100 | 73 |
| | | 150 | 134 |
| | | 175 | 188 |
| P 388 | SOAz | 200 | 4/10 guéries |
| | 3 injections Q4D | 50 | 73 |
| | | 100 | 196 |
| | 2 injections Q4D | 150 | 2/10 guéries |
| | 1 injection J + 1 | 50 | 33 |
| B 16 | SOAz | 100 | 47 |
| | | 150 | 64 |
| P 388 | 1 injection J + 1<br>SOPh | 200 | 113 |

On peut remarquer que les résultats essentiels de ce tableau sont les suivants:

Le composé $(NPAz_2)_2NSOAz$ guérit en monoinjection la leucémie P 388, ce qui est un fait rarissime pour ne pas dire exceptionnel au niveau de drogues anticancéreuses non-métalliques.

Le fait que l'ILS (196 %) obtenu en tri-injection Q4D 3 × 100 mg/kg soit pratiquement le triple de celui (73%) obtenu en injectant une fois 100 mg/kg au jour J + 1 permet de penser que ce composé est actif non seulement sur la leucémie P 388 précoce mais aussi sur cette leucémie à un stade avancé.

En outre, la figure ci-jointe met en évidence un phénomène semble-t-il unique en chimiothérapie du cancer. En effet, en mono-injection l'ILS du système composé/P 388 augmente exponentiellement avec la dose injectée, tendant vers la droite d'équation x = 200 mg/kg.

**Revendications pour les Etats contractants:**
**BE, CH, DE, FR, GB, IT, LI, LU, NL, SE**

1. Composés de formule:

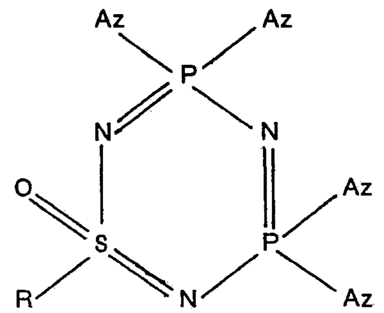

(I)

dans laquelle R est:
— un atome d'halogène,
— un radical alkyle, non substitué ou substitué,
— un radical alcényle, non substitué ou substitué,
— un radical alcynyle, non substitué ou substitué,
— un radical aryle, non substitué ou substitué,
— un radical alkoxy, non substitué ou substitué,
— un radical aryloxy, non substitué ou substitué,
— le radical hydroxy,
— le radical amino, un radical amino N-substitué ou N,N-disubstitué ou les substituants peuvent également former un cycle avec l'atome d'azote et Az est un radical 1-aziridinyle éventuellement substitué.

2. Composés selon la revendication 1, caractérisés en ce que le radical Az est le radical 1-aziridinyle 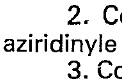.

3. Composés selon l'une des revendications 1 à 2, caractérisés en ce que R est un atome de fluor, de chlore, de brome, d'iode, un radical trifluorométhyle, phényle, diméthylamino, monométhylamino, aziridinyle.

4. Composés selon l'une des revendications 1 à 3, caractérisés en ce que R est le radical phényle.

5. Composés selon l'une des revendications 1 à 3, caractérisés en ce que R est le radical aziridinyle.

6. Composés selon l'une des revendications 1 à 3, caractérisés en ce que R est un atome de fluor.

7. Procédé de préparation des composés selon la revendication 1, caractérisé en ce que l'on fait réagir l'aziridine substituée ou non sur un composé de formule:

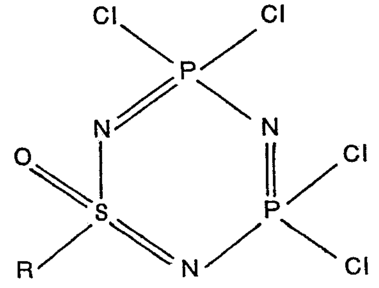

(II)

dans laquelle R a la signification donnée à la revendication 1, en proportion molaire d'au moins 4 moles d'aziridine substituée ou non par mole de composé II.

8. Procédé selon la revendication 7, caractérisé en ce que la réaction est conduite dans un solvant choisi parmi : les éthers, les hydrocarbures aliphatiques, les solvants aromatiques et les nitriles.

9. Procédé selon la revendication 8, caractérisé en ce que le solvant est le diéthyléther.

10. Procédé de préparation des composés de formule I dans lesquels R est un radical amino, amino mono- ou disubstitué, ou un radical alkoxy ou aryloxy, caractérisé en ce que l'on fait réagir un composé de formule:

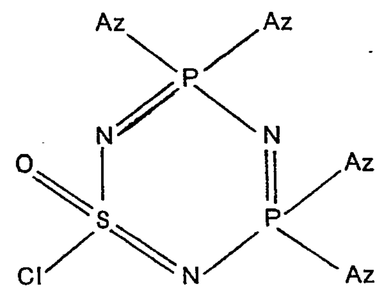

(III)

avec l'amine respectivement le dérivé hydroxylé correspondant au radical R.

11. Procédé selon la revendication 10, caractérisé en ce que le composé de formule III est préparé à partir du composé de formule IV $(NPCl_2)_2NSOCl$ par action de l'aziridine.

12. Procédé selon la revendication 11, pour la préparation du composé de formule I dans lequel R est le radical aziridinyle, caractérisé en ce que l'on fait réagir plus de 5 moles d'aziridine par mole de composé de formule IV sur le composé de formule IV $(NPCl_2)_2NSOCl$.

13. Procédé selon l'une des revendications 11 et 12, caractérisé en ce que le composé de formule III est préparé in situ et utilisé sans isolation.

14. Procédé de préparation du composé de formule I dans lequel R est le radical hydroxy, caractérisé en ce que l'on effectue l'hydrolyse d'un composé de formule:

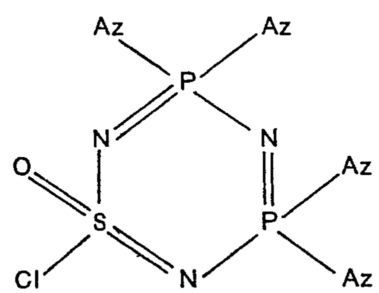

(III)

15. Composition pharmaceutique comprenant à titre de principe actif au moins un composé selon l'une des revendications 1 à 6.

16. Composition pharmaceutique selon la revendication 15, caractérisé en ce qu'elle se présente sous forme injectable.

17. Composition pharmaceutique selon la revendication 15, caractérisé en ce qu'elle se présente sous forme orale.

**Patentansprüche für die Vertragsstaaten: BE, CH, DE, FR, GB, IT, LI, LU, NL, SE**

1. Verbindungen der Formel

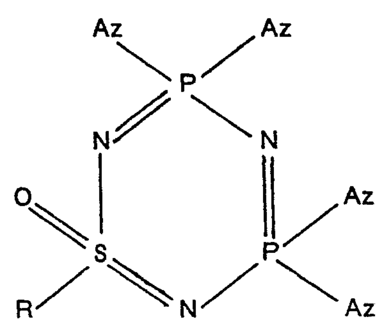

(I)

in welcher R

ein Halogenatom,
eine unsubstituierte oder substituierte Alkylgruppe,
eine unsubstituierte oder substituierte Alkenylgruppe,
eine unsubstituierte oder substituierte Alkinylgruppe,
eine unsubstituierte oder substituierte Arylgruppe,
eine unsubstituierte oder substituierte Alkoxygruppe,
eine unsubstituierte oder substituierte Aryloxygruppe,
die Hydroxygruppe,
eine Aminogruppe, eine N-substituierte oder N,N-disubstituierte Aminogruppe, in welcher die Substituenten mit dem Stickstoffatom einen Ring bilden können, bedeutet und Az einen gegebenenfalls substituierten 1-Aziridinylrest darstellt.

2. Verbindungen nach Anspruch 1, dadurch gekennzeichnet, daß Rest Az der 1-Aziridinylrest der Formel —N 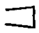 ist.

3. Verbindungen nach einem der Ansprüche 1 und 2, dadurch gekennzeichnet, daß R ein Fluoratom, ein Chloratom, ein Bromatom, ein Jodatom, eine Trifluormethylgruppe, eine Phenylgruppe, eine Dimethylaminogruppe, eine Monomethylaminogruppe oder eine Aziridinylegruppe ist.

4. Verbindungen nach einem der Ansprüche 1 bis 3, dadurch gekennzeichnet, daß R die Phenylgruppe ist.

5. Verbindungen nach einem der Ansprüche 1 bis 3, dadurch gekennzeichnet, daß R die Aziridinylgruppe ist.

6. Verbindungen nach einem der Ansprüche 1 bis 3, dadurch gekennzeichnet, daß R ein Fluoratom ist.

7. Verfahren zum Herstellen von Verbindungen gemäß Anspruch 1, dadurch gekennzeichnet, daß ein gegebenenfalls substituiertes Aziridin mit einer Verbindung der Formel

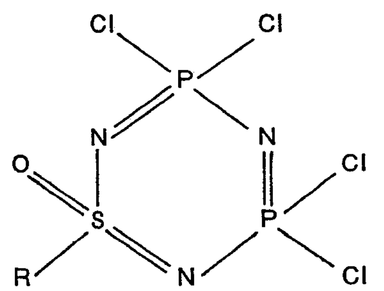

(II)

in welcher R die in Anspruch 1 angegebene Bedeutung besitzt, in einem Molverhältnis von zumindest 4 Mol des gegebenenfalls substituierten Aziridins je Mol der Verbindung (II) umgesetzt wird.

8. Verfahren nach Anspruch 7, dadurch gekennzeichnet, daß die Umsetzung in einem aus Äthern, aliphatischen Kohlenwasserstoffen, aromatischen Lösungsmitteln und Nitrilen ausgewählten Lösungsmittel durchgeführt wird.

9. Verfahren nach Anspruch 8, dadurch gekennzeichnet, daß als Lösungsmittel Diäthyläther verwendet wird.

10. Verfahren zum Herstellen von Verbindungen der Formel (I) in welchen R eine Aminogruppe, eine mono- oder disubstituierte Aminogruppe oder eine Alkoxygruppe oder eine Aryloxygruppe ist, dadurch gekennzeichnet, daß eine Verbindung der Formel

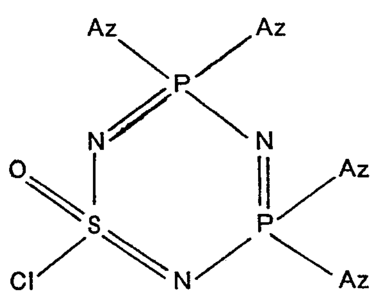

(III)

mit dem dem Rest R entsprechenden Amin- oder Hydroxyl-derivat umgesetzt wird.

11. Verfahren nach Anspruch 10, dadurch gekennzeichnet, daß die Verbindung der Formel (III) durch Umsetzen der Verbindung der Formel

$$(NPCl_2)_2NSOCl \qquad (IV)$$

mit Aziridin hergestellt wird.

12. Verfahren nach Anspruch 11, zum Herstellen der Verbindung der Formel (I) in welcher R die Aziridinylgruppe darstellt, dadurch gekennzeichnet, daß beim Umsetzen des Aziridins mit der Verbindung der Formel

$$(NPCl_2)_2NSOCl \qquad (IV)$$

mehr als 5 Mol Aziridin je Mol der Verbindung der Formel (IV) eingesetzt werden.

13. Verfahren nach Anspruch 11 oder 12, dadurch gekennzeichnet, daß die Verbindung der Formel (III) in Situ hergestellt und ohne Abtrennung verwendet wird.

14. Verfahren zum Herstellen der Verbindung der Formel (I) in welcher R die Hydroxygruppe darstellt, dadurch gekennzeichnet, daß eine Verbindung der Formel

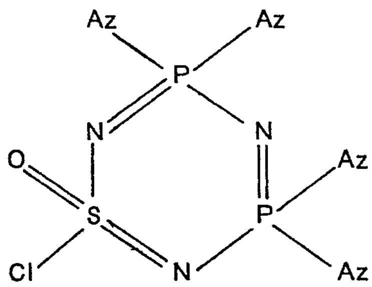
(III)

hydrolysiert wird.

15. Pharmazeutische Mischung, welche als Wirkstoff zumindest eine Verbindung gemäß einem der Ansprüche 1 bis 6 enthält.

16. Pharmazeutische Mischung nach Anspruch 15, dadurch gekennzeichnet, daß sie in injizierbarer Form vorliegt.

17. Pharmazeutische Mischung nach Anspruch 15, dadurch gekennzeichnet, daß sie in oral-verabreichbarer Form vorliegt.

**Claims for the Contracting States:**
**BE, CH, DE, FR, GB, IT, LI, LU, NL, SE**

1. Compounds corresponding to the following formula

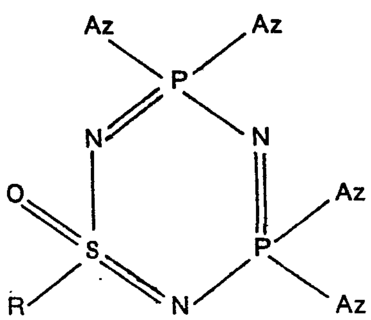
(I)

in which R is:
— a halogen atom,
— an unsubstituted or substituted alkyl radical,
— an unsubstituted or substituted alkenyl radical,
— an unsubstituted or substituted alkynyl radical,
— an unsubstituted or substituted aryl radical,
— an unsubstituted or substituted alkoxy radical,
— an unsubstituted or substituted aryloxy radical,
— the hydroxy radical, an amino radical,
— an N-substituted or N,N-disubstituted amino radical, wherein the substituants may built a cycle with the nitrogen atom, Az is an unsubstituted or substituted 1-aziridinyl radical.

2. Compounds as claimed in Claim 1, characterised in that the radical Az is the 1-aziridinyl radical 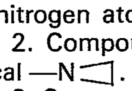.

3. Compounds as claimed in any of Claims 1 to 2, characterised in that R is a fluorine, chlorine, bromine, iodine atom, a trifluoromethyl, phenyl, dimethylamino, monomethylamino, aziridino radical.

4. Compounds as claimed in Claim 1 to 3, characterised in that R is the phenyl radical.

5. Compounds as claimed in Claim 1 to 3, characterised in that R is the aziridinyl radical.

6. Compounds as claimed in Claim 1 to 3, characterised in that R is a fluorine atom.

7. A process for the preparation of the compounds claimed in Claim 1, characterised in that the optionally substituted aziridine is reacted with a compound corresponding to the following formula

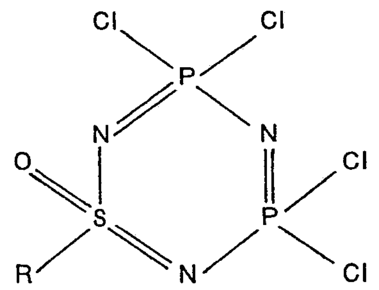

(II)

in which R is as defined in claim 1 the molar ratio being at least four moles of optionally substituted aziridine by mole of compound II.

8. A process as claimed in Claim 7, characterised in that the reaction is carried out in a solvent selected from ethers, aliphatic hydrocarbons, aromatic solvents and nitriles.

9. A process as claimed in Claim 8, characterised in that the solvent is diethylether.

10. A process for the preparation of compounds corresponding to formula I in which R is an amino mono- or di-substituted amino radical or an aryloxy or alkoxy radical, characterised in that a compound corresponding to the following formula

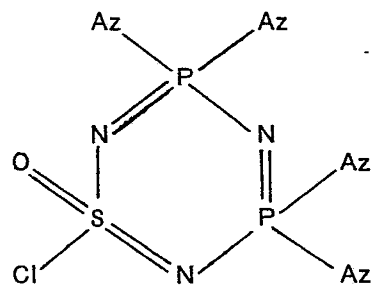

(III)

is reacted with the amine respectively the hydroxy derivative corresponding to the radical R.

11. A process as claimed in Claim 10, characterised in that the compound of formula III is prepared from the compound of formula IV $(NPCl_2)_2NSOCl$ by the action of aziridine.

12. A process as claimed in Claim 11 for the preparation of the compound of formula I in which R is the aziridinyl radical, characterised in that the aziridine is reacted with more than 5 moles of aziridine by mole of compound IV with the compound of formula IV $(NPCl_2)_2NSOCl$.

13. A process as claimed in Claims 11 or 12, characterised in that compound III is prepared in situ and reacted without isolation.

14. A process for the preparation of the compound of formula I in which R is the hydroxy radical, characterised in that a compound corresponding to the following formula

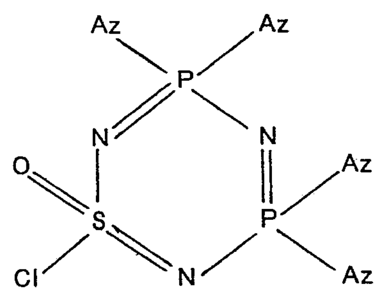

(III)

is subjected to hydrolysis.

15. A pharmaceutical composition containing as active principle at least one compound of the type claimed in any of claims 1 to 6.

16. A pharmaceutical composition as claimed in Claim 15, characterised in that it is made up in injectable form.

17. A pharmaceutical composition as claimed in Claim 15, characterised in that it is made up in oral form.

**Revendications pour l'Etat contractant:**
**AT**

1. Procédé de préparation des composés de formule:

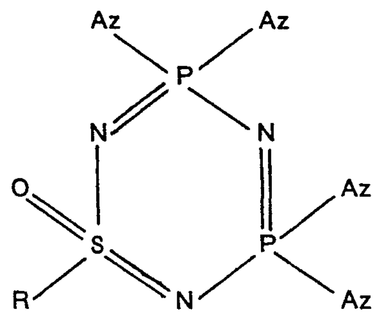

(I)

dans laquelle R est:
— un atome d'halogène,
— un radical alkyle, non substitué ou substitué
— un radical alcényle, non substitué ou substitué
— un radical alcynyle, non substitué ou substitué
— un radical aryle, non substitué ou substitué
— un radical alkoxy, non substitué ou substitué
— un radical aryloxy, non substitué ou substitué
— le radical hydroxy,
— le radical amino, un radical amino N-substitué ou N,N-disubstitué ou les substituants peuvent également former un cycle avec l'atome d'azote et Az est un radical 1-aziridinyle éventuellement substitué, caractérisé en ce que l'on fait réagir l'aziridine substitué ou non sur un composé de formule:

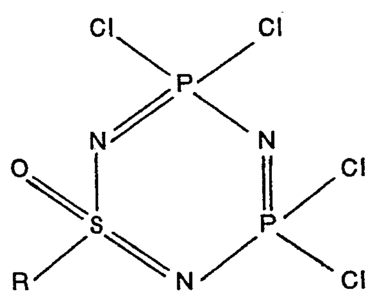

(II)

dans laquelle R a la signification donnée précédemment, en proportion molaire d'au moins 4 moles d'aziridine substitué ou non par mole de composé II.

2. Procédé selon la revendication 1, caractérisé en ce que la réaction est conduite dans un solvant choisi parmi : les éthers, les hydrocarbures aliphatiques, les solvants aromatiques et les nitriles.

3. Procédé selon la revendication 2, caractérisé en ce que le solvant est le diéthyléther.

4. Procédé selon l'une des revendications 1 à 3, caractérisé en ce que le radical Az est le radical 1-aziridinyle 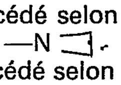.

5. Procédé selon l'une des revendications 1 à 4, caractérisé en ce que R est un atome de fluor, de chlore, de brome, d'iode, un radical trifluorométhyle, phényle, diméthylamino, monométhylamino, aziridinyle.

6. Procédé selon l'une des revendications 1 à 4, caractérisé en ce que R est le radical phényle.

7. Procédé selon l'une des revendications 1 à 3, caractérisé en ce que R est le radical aziridinyle.

8. Procédé selon l'une des revendications 1 à 3, caractérisé en ce que R est un atome de fluor.

9. Procédé de préparation des composés de formule I dans lesquels R est un radical amino,

amino mono- ou disubstitué, ou un radical alkoxy ou aryloxy, caractérisé en ce que l'on fait réagir un composé de formule:

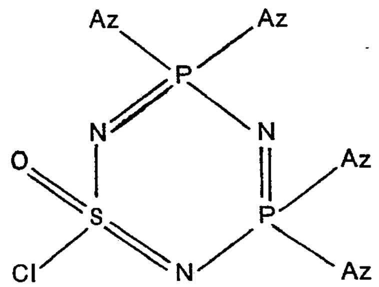

(III)

avec l'amine respectivement le dérivé hydroxylé correspondant au radical R.

10. Procédé selon la revendication 9, caractérisé en ce que le composé de formule III est préparé à partir du composé de formule IV $(NPCl_2)_2NSOCl$ par action de l'aziridine.

11. Procédé selon la revendication 10, pour la préparation du composé de formule I dans lequel R est le radical aziridinyle, caractérisé en ce que l'on fait réagir plus de 5 moles d'aziridine par mole de composé de formule IV sur le composé de formule IV $(NPCl_2)_2NSOCl$.

12. Procédé selon l'une des revendications 10 et 11, caractérisé en ce que le composé de formule III est préparé in situ et utilisé sans isolation.

13. Procédé de préparation du composé de formule I dans lequel R est le radical hydroxy, caractérisé en ce que l'on effectue l'hydrolyse d'un composé de formule:

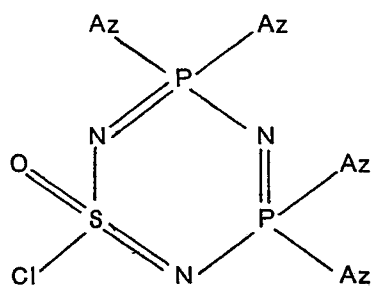

(III)

**Patentansprüche für den Vertragsstaat: AT**

1. Verfahren zum Herstellen von Verbindungen der Formel

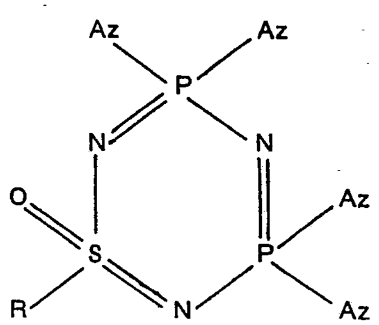

(I)

in welcher R
ein Halogenatom,
eine unsubstituierte oder substituierte Alkylgruppe,
eine unsubstituierte oder substituierte Alkenylgruppe,
eine unsubstituierte oder substituierte Alkinylgruppe,
eine unsubstituierte oder substituierte Arylgruppe,
eine unsubstituierte oder substituierte Alkoxygruppe,
eine unsubstituierte oder substituierte Aryloxygruppe,
die Hydroxygruppe,
eine Aminogruppe, eine N-substituierte oder N,N-disubstituierte Aminogruppe, in welcher die Substituenten mit dem Stickstoffatom einen Ring bilden können, bedeutet und Az einen gegebenenfalls

substituierten 1-Aziridinylrest darstellt, dadurch gekennzeichnet, daß ein gegebenenfalls substituiertes Aziridin mit einer Verbindung der Formel

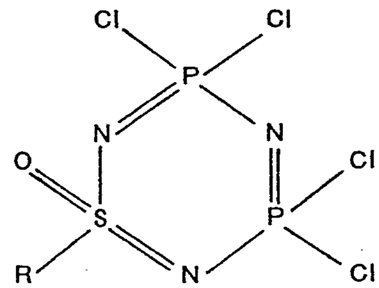

(II)

in welcher R die in Anspruch 1 angegebene Bedeutung besitzt, in einem Molverhältnis von zumindest 4 Mol des gegebenenfalls substituierten Aziridins je Mol der Verbindung (II) umgesetzt wird.

2. Verfahren nach Anspruch 1, dadurch gekennzeichnet, daß die Umsetzung in einem aus Äthern, aliphatischen Kohlenwasserstoffen, aromatischen Lösungsmitteln und Nitrilen ausgewählten Lösungsmittel durchgeführt wird.

3. Verfahren nach Anspruch 2, dadurch gekennzeichnet, daß als Lösungsmittel Diäthyläther verwendet wird.

4. Verfahren nach einem der Ansprüche 1 bis 3, dadurch gekennzeichnet, daß Rest Az der 1-Aziridinylrest der Formel —N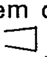 ist.

5. Verfahren nach einem der Ansprüche 1 bis 4, dadurch gekennzeichnet, daß R ein Fluoratom, ein Chloratom, ein Bromatom, ein Jodatom, eine Trifluormethylgruppe, eine Phenylgruppe, eine Dimethylaminogruppe, eine Monomethylaminogruppe oder eine Aziridinylgruppe ist.

6. Verfahren nach einem der Ansprüche 1 bis 4, dadurch gekennzeichnet, daß R die Phenylgruppe ist.

7. Verfahren nach einem der Ansprüche 1 bis 3, dadurch gekennzeichnet, daß R die Aziridinylgruppe ist.

8. Verfahren nach einem der Ansprüche 1 bis 3, dadurch gekennzeichnet, daß R ein Fluoratom ist.

9. Verfahren zum Herstellen von Verbindungen der Formel (I) in welchen R eine Aminogruppe, eine mono- oder disubstituierte Aminogruppe oder eine Alkoxygruppe oder eine Aryloxygruppe ist, dadurch gekennzeichnet, daß eine Verbindung der Formel

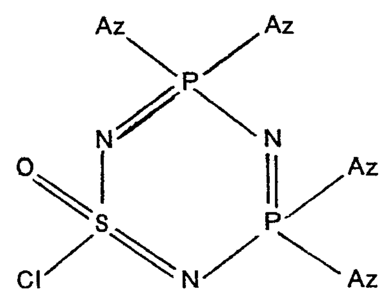

(III)

mit dem dem Rest R entsprechenden Amin- oder Hydroxyl-derivat umgesetzt wird.

10. Verfahren nach Anspruch 9, dadurch gekennzeichnet, daß die Verbindung der Formel (III) durch Umsetzen der Verbindung der Formel

$$(NPCl_2)_2NSOCl \qquad (IV)$$

mit Aziridin hergestellt wird.

11. Verfahren nach Anspruch 10, zum Herstellen der Verbindung der Formel (I) in welcher R die Aziridinylgruppe darstellt, dadurch gekennzeichnet, daß beim Umsetzen des Aziridins mit der Verbindung der Formel

$$(NPCl_2)_2NSOCl \qquad (IV)$$

mehr als 5 Mol Aziridin je Mol der Verbindung der Formel (IV) eingesetzt werden.

12. Verfahren nach Anspruch 10 oder 11, dadurch gekennzeichnet, daß die Verbindung der Formel (III) in Situ hergestellt und ohne Abtrennung verwendet wird.

13. Verfahren zum Herstellen der Verbindung der Formel (I) in welcher R die Hydroxygruppe darstellt, dadurch gekennzeichnet, daß eine Verbindung der Formel

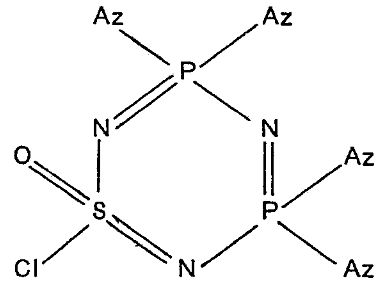

(III)

hydrolysiert wird.

**Claims for the Contracting State: AT**

1. A process for the preparation of the compounds corresponding to the following formula

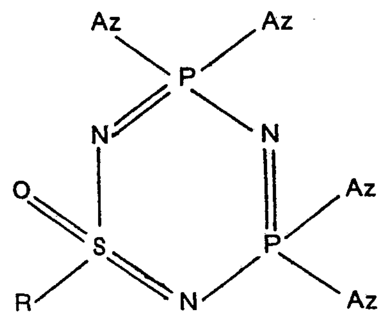

(I)

in which R is:
— a halogen atom,
— an unsubstituted or substituted alkyl radical,
— an unsubstituted or substituted alkenyl radical,
— an unsubstituted or substituted alkynyl radical,
— an unsubstituted or substituted aryl radical,
— an unsubstituted or substituted alkoxy radical,
— an unsubstituted or substituted aryloxy radical,
— the hydroxy radical, an amino radical,
— an N-substituted or N,N-disubstituted amino radical,
wherein the substituants may built a cycle with the nitrogen atom, Az is an unsubstituted or substituted 1-aziridinyl radical, characterised in that the optionally substituted aziridine is reacted with a compound corresponding to the following formula

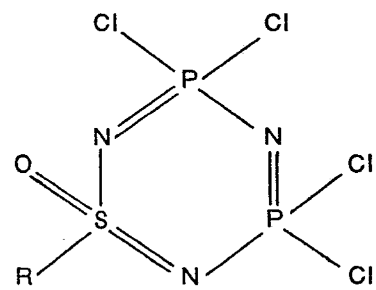

(II)

in which R is as defined in claim 1 the molar ratio being at least four moles of optionally substituted aziridine by mole of compound II.

2. A process as claimed in Claim 1, characterised in that the reaction is carried out in a solvent selected from ethers, aliphatic hydrocarbons, aromatic solvents and nitriles.

3. A process as claimed in Claim 2, characterised in that the solvent is diethylether.

4. A process as claimed in any of claims 1 to 3, characterised in that the radical Az is the 1-aziridinyl radical 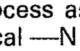.

5. A process as claimed in any of claims 1 to 4, characterised in that R is a fluorine, chlorine, bromine, iodine atom, a trifluoromethyl, phenyl, dimethylamino, monomethylamino, aziridino radical.

6. A process as claimed in any of claims 1 to 4, characterised in that R is the phenyl radical.

7. A process as claimed in any of claims 1 to 3, characterised in that R is the aziridinyl radical.

8. A process as claimed in any of claims 1 to 3, characterised in that R is a fluorine atom.

9. A process for the preparation of compounds corresponding to formula I in which R is an amino mono- or di-substituted amino radical or an aryloxy or alkoxy radical, characterised in that a compound corresponding to the following formula

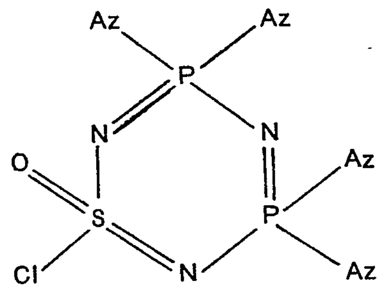

(III)

is reacted with the amine respectively the hydroxy derivative corresponding to the radical R.

10. A process as claimed in Claim 9, characterised in that the compound of formula III is prepared from the compound of formula IV $(NPCl_2)_2NSOCl$ by the action of aziridine.

11. A process as claimed in Claim 10 for the preparation of the compound of formula I in which R is the aziridinyl radical, characterised in that the aziridine is reacted with more than 5 moles of aziridine by mole of compound IV with the compound of formula IV $(NPCl_2)_2NSOCl$.

12. A process as claimed in Claim 10 or 11, characterised in that compound III is prepared in situ and reacted without isolation.

13. A process for the preparation of the compound of formula I in which R is the hydroxy radical, characterised in that a compound corresponding to the following formula

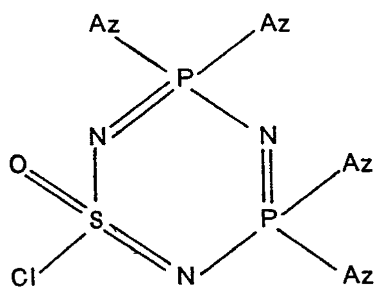

(III)

is subjected to hydrolysis.

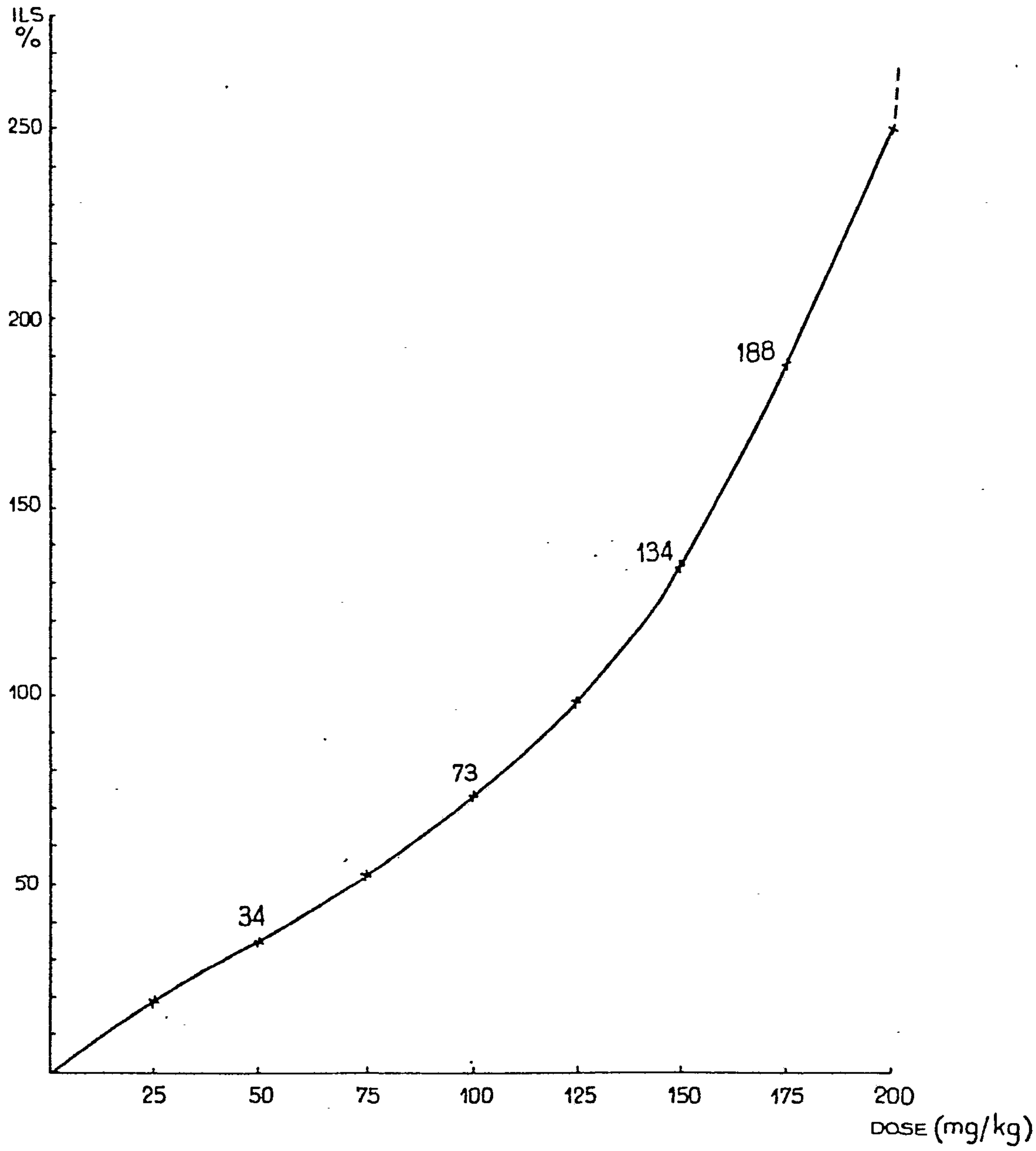
ILS
%
250
200
150
100
50
25
50
75
100
125
150
175
200
DOSE (mg/kg)
34
73
134
188

SOAz
(P 388)